(19)

Europäisches
Patentamt
European
Patent Office
Office européen
des brevets

(11) **EP 4 696 272 A1**

(12) **EUROPEAN PATENT APPLICATION**
published in accordance with Art. 153(4) EPC

(43) Date of publication:
**18.02.2026 Bulletin 2026/08**

(21) Application number: **23932689.5**

(22) Date of filing: **22.08.2023**

(51) International Patent Classification (IPC):
***A61B 34/30*** (2016.01)

(86) International application number:
**PCT/CN2023/114248**

(87) International publication number:
**WO 2024/212416 (17.10.2024 Gazette 2024/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.04.2023 CN 202310395744**

(71) Applicant: **Hanglok-Tech Co., Ltd.**
**Zhuhai, Guangdong 519090 (CN)**

(72) Inventors:
• **WANG, Cheng**
**Zhuhai, Guangdong 519090 (CN)**
• **CHEN, Shijia**
**Zhuhai, Guangdong 519090 (CN)**
• **ZHANG, Yongcheng**
**Zhuhai, Guangdong 519090 (CN)**
• **CHEN, Xiaodong**
**Zhuhai, Guangdong 519090 (CN)**
• **LUO, Hao**
**Zhuhai, Guangdong 519090 (CN)**
• **LI, Chichi**
**Zhuhai, Guangdong 519090 (CN)**
• **CHEN, Zexin**
**Zhuhai, Guangdong 519090 (CN)**

(74) Representative: **Bratovic, Nino Maria**
**Bratovic IP**
**Lissi-Kaeser-Straße 20**
**80797 München (DE)**

(54) **PUNCTURE SURGICAL ROBOT AND CONTROL METHOD THEREFOR, CONTROLLER, AND STORAGE MEDIUM**

(57) A puncture operation robot and a control method therefor are disclosed. The control method includes: obtaining, by a force sensing module, a first axial force on an ultrasonic probe; calculating a second axial force on the ultrasonic probe along a central axial direction according to the first axial force and a preset force perception model; calibrating, by an optical positioning acquisition module, a normal vector of a contact point between the ultrasonic probe and skin; a normal pressure of the ultrasonic probe on the skin is calculated according to the second axial force and the normal vector; in response to the normal pressure being greater than or equal to a force criterion threshold, controlling a mechanical arm to stop pressing down; and displaying, by a display module in real time, images of an area to be punctured acquired by an ultrasonic image perception module and the optical positioning acquisition module.

obtain, by means of a force sensing module, a first axial force on an ultrasonic probe — S110

calculate a second axial force on the ultrasonic probe along a central axial direction according to the first axial force and a preset force perception model — S120

calibrate, by means of an optical positioning acquisition module, a normal vector of a contact point between the ultrasonic probe and skin — S130

perform calculation processing according to the second axial force and the normal vector to obtain a normal pressure of the ultrasonic probe on the skin — S140

in response to the normal pressure being greater than or equal to a force criterion threshold, control a mechanical arm to stop pressing down — S150

display, by a display module in real time, images of an area to be punctured acquired by an ultrasonic image perception module and the optical positioning acquisition module — S160

FIG. 7

EP 4 696 272 A1

## Description

## TECHNICAL FIELD

The present disclosure relates to the technical field of medical device, and in particular, to a puncture operation robot and a control method therefor, a controller, and a storage medium.

## BACKGROUND

With the advancement of robotics and the innovation of medical technology, the combination of medicine and robotics has become one of the development trends in the medical industry. Medical robots refer to various robotic devices used in surgical operations, medical training, rehabilitation therapy, prostheses, and assistance for the disabled. Puncture operation robots are one of the branches of medical surgical robots. Puncture approaches of the existing puncture operation robots can be generally divided into fully automatic puncture and semi-automatic puncture. Specifically, fully automatic puncture means that a needle is directly fixed on the distal end of a mechanical arm, and the robot is manipulated all the way to thrust the needle into a patient's body to achieve a puncture function. This has a low sense of doctor participation and a high risk factor. Semi-automatic puncture means that the robot mainly plays a positioning function during an operation, and a final puncture action still requires participation of a doctor. Compared with fully automatic puncture, semi-automatic puncture allows the doctor to have a stronger sense of participation and has a lower risk factor. However, when using the puncture operation robot for semi-automatic puncture, it is difficult to control a fitting pressure when an ultrasonic probe is fitted to a human body. This can easily lead to a situation where the fitting pressure is too low, resulting in blurred ultrasonic images, or where the pressure is too high, which needs to be prevented as it causes severe squeezing and deformation of organs. It is difficult to ensure the operation accuracy and safety when using the puncture operation robot for a puncture operation.

## SUMMARY

The following is a summary of subject matters described in detail herein.

In accordance with an embodiment of the present disclosure, provided are a puncture operation robot and a control method therefor, a controller, and a storage medium, which can provide doctors with reliable operation reference information by utilizing the puncture operation robot, thereby improving the operation efficiency, operation accuracy and safety when a semi-automatic puncture operation is performed by using the puncture operation robot.

In accordance with a first aspect of the present disclosure, an embodiment provides a puncture operation robot, which includes:

a mechanical arm;

a puncture mechanism arranged at a distal end of the mechanical arm;

a puncture perception device arranged on the puncture mechanism, the puncture perception device includes a display module, an optical positioning acquisition module, a force sensing module, and an ultrasonic image perception module including an ultrasonic probe; and

a controller, where the controller is electrically connected to the mechanical arm, the display module, the optical positioning acquisition module, the ultrasonic image perception module and the force sensing module, respectively.

In accordance with some embodiments of the present disclosure, the force sensing module includes at least two one-dimensional sensors.

In accordance with a second aspect of the present disclosure, an embodiment provides a method for controlling a puncture operation robot, applied to the puncture operation robot as described in the first aspect, where the puncture operation robot includes: a mechanical arm; a puncture mechanism arranged at a distal end of the mechanical arm; a puncture perception device arranged on the puncture mechanism, the puncture perception device includes a display module, an optical positioning acquisition module, a force sensing module, and an ultrasonic image perception module including an ultrasonic probe; and a controller, where the controller is electrically connected to the mechanical arm, the display module, the optical positioning acquisition module, the ultrasonic image perception module and the force sensing module, respectively; and
where the control method includes:

obtaining, by the force sensing module, a first axial force on the ultrasonic probe;

calculating a second axial force on the ultrasonic probe along a central axial direction based on the first axial force and a preset force perception model;

calibrating, by the optical positioning acquisition module, a normal vector of a contact point between the ultrasonic probe and skin;

performing calculation processing based on the second axial force and the normal vector to obtain a normal pressure of the ultrasonic probe on the skin;

in response to the normal pressure being greater than or equal to a force criterion threshold, controlling the mechanical arm to stop pressing down; and

displaying, by the display module in real time, images of an area to be punctured acquired by the ultrasonic image perception module and the optical positioning acquisition module.

In accordance with some embodiments of the present disclosure, the force sensing module includes at least two one-dimensional sensors; the obtaining, by the force sensing module, a first axial force on the ultrasonic probe includes:

obtaining, by the at least two one-dimensional sensors, at least two axial force components; and

obtaining, by summing the at least two axial force components, the first axial force.

In accordance with some embodiments of the present disclosure, the calculating a second axial force on the ultrasonic probe along a central axial direction based on the first axial force and a preset force perception model includes:

setting the preset force perception model to be $\vec{f}_s = k * \vec{F}_1$, where $\vec{F}_1$ is the first axial force, $\vec{f}_s$ is the second axial force, and k is a constant coefficient; and

inputting the measured first axial force into the preset force perception model for calculation to obtain the second axial force.

In accordance with some embodiments of the present disclosure, the performing calculation processing based on the second axial force and the normal vector to obtain a normal pressure of the ultrasonic probe on the skin includes:

determining a first angle between the second axial force and the normal vector; and

obtaining a normal pressure of the ultrasonic probe on the skin by performing calculation based on the second axial force and a cosine value of the first angle.

In accordance with some embodiments of the present disclosure, the images of the area to be punctured include a lesion image and a position reference image; and the displaying, by the display module in real time, images of an area to be punctured acquired by the ultrasonic image perception module and the optical positioning acquisition module includes:

acquiring an ultrasonic image of the skin through the ultrasonic image perception module;

performing image processing on the ultrasonic image to obtain the lesion image;

obtaining a needle thrusting path of a puncture distal end of the puncture mechanism through the optical positioning acquisition module; and

displaying, by the display module in real time, the lesion image and the position reference image, the position reference image is used for displaying a positional relationship between the needle thrusting path and a lesion on the skin.

In accordance with some embodiments of the present disclosure, the control method further includes: when the normal pressure is smaller than the force criterion threshold, controlling the mechanical arm to keep pressing down.

In accordance with a third aspect of the present disclosure, an embodiment provides a controller, which includes: a memory, a processor, and a computer program stored in the memory and executable by the processor, where the computer program, when executed by the processor, causes the processor to perform the method for controlling the puncture operation robot as described in the second aspect.

In accordance with a fourth aspect of the present disclosure, an embodiment provides a computer-readable storage medium storing computer-executable instructions, where the computer-executable instructions are configured to enable a computer to execute the method for controlling the puncture operation robot as described in the second aspect.

Embodiments of the present disclosure include: During a semi-automatic puncture operation, the puncture operation robot first obtains, by the force sensing module, the first axial force on the ultrasonic probe; next, the second axial force on the ultrasonic probe along the central axial direction is calculated according to the first axial force and the preset force perception model; then, the normal vector of the contact point between the ultrasonic probe and the skin is calibrated by the optical positioning acquisition module; next, calculation processing is performed according to the second axial force and the normal vector to obtain the normal pressure of the ultrasonic probe on the skin; finally, in response to the normal pressure being greater than or equal to the force criterion threshold, the mechanical arm is controlled to stop pressing down, thereby prompting a doctor that the normal pressure generated by fitting of the ultrasonic probe and the patient's skin is appropriate, which effectively reduces the probability of two situations: the normal pressure of fitting is too small, causing blurred ultrasonic images; and the pressure is too large, causing severe compression and deformation of the patient's organs. Simultaneously, the images of the area to be punctured that are acquired by the ultrasonic image perception module and the optical positioning acquisition module are displayed by the display module in real time, so that the doctor can complete a puncture action by adjusting and operating the puncture mechanism based on the displayed images, thus achieving the semi-automatic puncture operation with enhanced efficiency, accuracy, and safety. That is to say, the embodiments of the present disclosure can provide doctors with reliable operation reference information by utilizing the puncture operation robot, thereby improving the operation efficiency, operation accuracy and safety when the semi-automatic puncture operation is performed by using the puncture operation robot.

## BRIEF DESCRIPTION OF DRAWINGS

FIG. 1 is a schematic diagram of a system architecture of a puncture operation robot 100 for executing a control method according to an embodiment of the present disclosure;

FIG. 2 is a front view of a puncture perception device according to an embodiment of the present disclosure;

FIG. 3 is a side view of a puncture perception device according to an embodiment of the present disclosure;

FIG. 4 is a side section view of a puncture perception device according to an embodiment of the present disclosure;

FIG. 5 is a schematic diagram of a force sensing module according to an embodiment of the present disclosure;

FIG. 6 is a schematic diagram of a force sensing module according to another embodiment of the present disclosure;

FIG. 7 is a flow chart of a method for controlling a puncture operation robot according to an embodiment of the present disclosure;

FIG. 8 is a schematic diagram of using a discrete method for an arc curved surface at the distal end of an ultrasonic probe according to an embodiment of the present disclosure;

FIG. 9 is a flow chart of a specific method of step S140 in FIG. 7;

FIG. 10 is a schematic diagram of force application when an ultrasonic probe fits human skin according to an embodiment of the present disclosure;

FIG. 11 is a schematic diagram of force application when an ultrasonic probe moves along a needle thrusting direction according to an embodiment of the present disclosure;

FIG. 12 is a schematic diagram of force application when an ultrasonic probe moves along a distal axial direction according to an embodiment of the present disclosure;

FIG. 13 is a schematic diagram of force application when an ultrasonic probe fits human skin according to another embodiment of the present disclosure;

FIG. 14 is a statistical distribution diagram of quantity-interval values according to an embodiment of the present disclosure; and

FIG. 15 is a schematic diagram of a hardware structure of a controller according to an embodiment of the present disclosure.

## DETAILED DESCRIPTION

In order to make the objectives, technical solutions and advantages of the present disclosure more clearly understood, the present disclosure is further described in detail below with reference to the accompanying drawings and embodiments.

It should be noted that although a logical order is shown in the flow chart, in some cases, the steps shown or described may be performed in an order different from that in the flow chart. The terms "first", "second" and the like in the specification, claims and drawings are used to distinguish similar objects and are not necessarily used to describe a particular order or sequence.

Unless defined otherwise, all technical and scientific terms used herein have the same meaning as commonly understood by those having ordinary skill in the art to which this application belongs. The terms used herein are only for the purpose of describing the embodiments of the present application and are not intended to limit the present application.

First, some of the terms used in this application are explained as follows.

Optical positioning principles: For example, by capturing near-infrared light actively emitted from the same marker from different angles, three-dimensional spatial coordinates of each marker at different times can be obtained in real time and accurately through relevant calculations and analysis.

Embodiments of the present disclosure provide a puncture operation robot and a control method therefor, a controller, and a computer-readable storage medium; during a semi-automatic puncture operation, the puncture operation robot first obtains, by means of a force sensing module, a first axial force on an ultrasonic probe; next, a second axial force on the ultrasonic probe along a central axial direction is calculated according to the first axial force and a preset force perception model; then, a normal vector of a contact point between the ultrasonic probe and skin is calibrated by means of an optical positioning acquisition module; next, calculation processing is performed according to the second axial force and the normal vector to obtain a normal pressure of the ultrasonic probe on the skin; finally, in response to the normal pressure being greater than or equal to a force criterion threshold, a mechanical arm is controlled to stop pressing down; simultaneously, images of an area to be punctured that are acquired by an ultrasonic image perception module and the optical positioning acquisition module are displayed by means of a display module in real time. Hence, the embodiments of the present disclosure can provide doctors with reliable operation reference information by utilizing the puncture operation robot, thereby improving the operation efficiency, operation accuracy and safety when the semi-automatic puncture operation is performed by using the puncture operation robot.

The embodiments of the present disclosure are further described below with reference to the accompanying drawings.

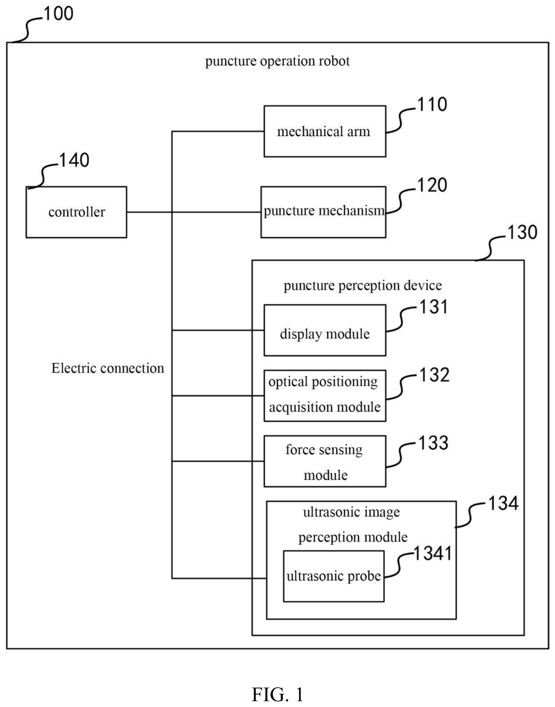

As shown in FIG. 1, the puncture operation robot 100 includes: a mechanical arm 110; a puncture mechanism 120 arranged at a distal end of the mechanical arm 110; a puncture perception device 130 arranged on the puncture mechanism 120, the puncture perception device 130 includes a display module 131, an optical positioning acquisition module 132, a force sensing module 133, and an ultrasonic image perception module 134 including an ultrasonic probe 1341; and a controller 140, the controller 140 is electrically connected to the mechanical arm 110, the display module 131, the optical positioning acquisition module 132, the ultrasonic image perception module 134 and the force sensing module 133, respectively.

The puncture mechanism 120 is used to perform a puncture action.

The puncture perception device 130 includes the display module 131, the optical positioning acquisition module 132, the force sensing module 133, and the ultrasonic image perception module 134 including the ultrasonic probe 1341. The display module 131 is used for displaying real-time images of an area to be punctured. The optical positioning acquisition module 132 is used to achieve optical positioning. The force sensing module 133 is used to output a force feedback signal. The ultrasonic image perception module 134 is used to acquire ultrasonic images of skin. By using the puncture sensing module, mixed perception of forces, spatial positions, and real-time images can be achieved in a semi-automatic puncture operation.

During the semi-automatic puncture operation, the controller 140 is used to obtain, by means of the force sensing module 133, a first axial force on the ultrasonic probe 1341; next, a second axial force on the ultrasonic probe 1341 along a central axial direction is calculated according to the first axial force and a preset force perception model; then, a normal vector of a contact point between the ultrasonic probe 1341 and skin is calibrated by means of the optical positioning acquisition module 132; next, calculation processing is performed according to the second axial force and the normal vector to obtain a normal pressure of the ultrasonic probe 1341 on the skin; finally, in response to the normal pressure being greater than or equal to a force criterion threshold, the mechanical arm 110 is controlled to stop pressing down, thereby prompting a doctor that the normal pressure generated by fitting of the ultrasonic probe 1341 and the patient's skin is appropriate, which effectively reduces the probability of two situations: the normal pressure of fitting is too small, causing blurred ultrasonic images; and the pressure is too large, causing severe compression and deformation of the patient's organs. Simultaneously, images of an area to be punctured that are acquired by the ultrasonic image perception module 134 and the optical positioning acquisition module 132 are displayed by means of the display module in real time, so that the doctor can complete a puncture action by adjusting and operating the puncture mechanism 120 based on the displayed images, thus achieving the semi-automatic puncture operation with enhanced efficiency, accuracy, and safety.

In accordance with the embodiments provided by the present disclosure, doctors may be provided with reliable operation reference information by utilizing the puncture operation robot 100, thereby improving the operation efficiency, operation accuracy and safety when the semi-automatic puncture operation is performed by using the puncture operation robot 100.

Based on the above system architecture, various embodiments of the puncture perception device 130 of the present disclosure are proposed.

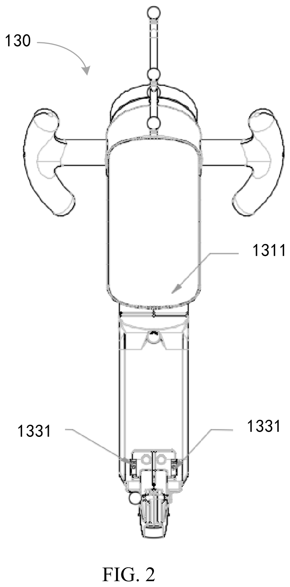

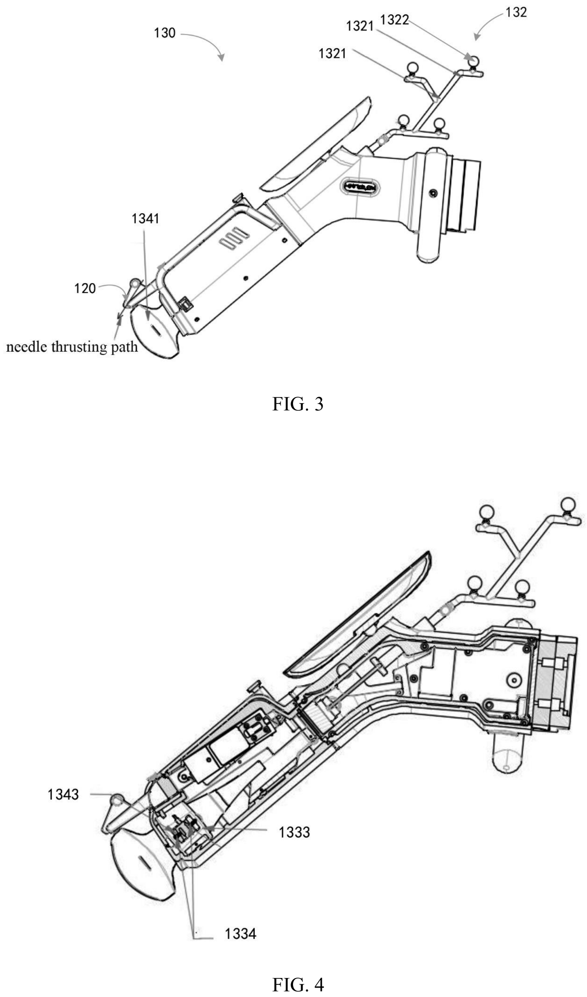

As shown in FIGS. 1-4 , the puncture perception device 130 includes the display module 131, the optical positioning acquisition module 132, the force sensing module 133 and the ultrasonic image perception module 134.

The ultrasonic image perception module 134 includes the ultrasonic probe 1341 and an ultrasonic probe fixing clip 1342. The ultrasonic probe 1341 and the ultrasonic probe fixing clip 1342 are connected, and the ultrasonic probe fixing clip 1342 is used to fix and install the ultrasonic probe 1341. The ultrasonic probe 1341 is used to fit the patient's skin to obtain ultrasonic images of the skin, so that the controller 140 performs image processing on the ultrasonic images based on a software algorithm to obtain a lesion image.

The optical positioning acquisition module 132 includes small ball seats 1321 and optical positioning small balls 1322. The small ball seat 1321 is used to place the optical positioning small ball 1322, and the small ball seat 1321 and the optical positioning small ball 1322 are connected. The optical positioning small ball 1322 is used to acquire spatial coordinates of the distal end of the ultrasonic probe 1341, so that the controller 140 can perform coordinate processing based on the optical positioning principle to obtain a motion path of the puncture distal end and a needle thrusting path. In one embodiment, the motion path refers to the movement direction of the ultrasonic probe 1341 when being moved, which can be along an axis of its distal end or along a needle thrusting direction. The needle thrusting path refers to a direction of a needle thrusting hole of the puncture mechanism 120. In one embodiment, there are four optical positioning small balls 1322. It is understandable that the number of optical positioning small balls 1322 may be set according to actual needs, and the present disclosure does not impose any specific limitation on this.

The display module includes a display screen 1311, which is in communication connection with the controller 140 and may display the lesion image and the position reference image acquired by the ultrasonic image perception module 134 under the control of the controller 140, where the position reference image is used for displaying a positional relationship between the needle thrusting path and a lesion on the skin, which allows the doctor to observe the positional relationship between the needle thrusting path and the lesion in real time on the display screen 1311 when thrusting the needle, thereby improving the operation efficiency, operation accuracy and safety of the puncture operation. It is understandable that the display module includes one display screen 1311 or multiple display screens 1311. When there are multiple display screens 1311, a plurality of them may be set independently from the puncture operation robot 100, which is conducive to multi-party consultation and surgical detection.

The force sensing module 133 includes two one-dimensional sensors 1331. The two one-dimensional sensors 1331 are respectively disposed on both sides of the ultrasonic probe 1341. In one embodiment, the force sensing module 133 further includes an end mounting bracket 1333 and a fixing screw 1334. One end of the ultrasonic probe fixing clip 1342 is connected to the ultrasonic probe 1341, and another end of the ultrasonic probe fixing clip 1342 is connected to an acquisition end of each of the two one-dimensional sensors 1331. A fixed end of each of the two one-dimensional sensors 1331 is connected to the end mounting bracket 1333 and fixed by the fixing screw 1334. When the ultrasonic probe 1341 fits the human body, the two one-dimensional sensors 1331 may dynamically measure two axial force components along the axial direction on both sides of the ultrasonic probe 1341, so that the controller 140 may calculate the first axial force based on the two axial force components, and then calculate the second axial force along the central axial direction of the ultrasonic probe 1341 according to the first axial force and the preset force perception model, and finally calculate the normal pressure of the ultrasonic probe 1341 on the skin based on the second axial force, so as to judge in real time whether the degree of fitting between the probe and the human body is reasonable through the normal pressure and the force criterion threshold.

In accordance with some embodiments of the present disclosure, the force sensing module 133 includes at least two one-dimensional sensors 1331. As shown in FIG. 1, the force sensing module 133 includes two one-dimensional sensors 1331.

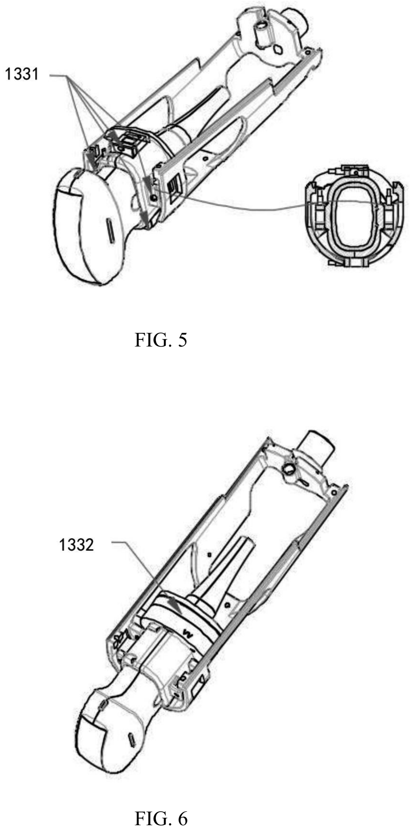

In addition, as shown in FIG. 5, the force sensing module 133 includes four one-dimensional sensors 1331, that is, two one-dimensional sensors 1331 are respectively arranged on both sides of the ultrasonic probe 1341. When the ultrasonic probe 1341 fits the human body, the four one-dimensional sensors 1331 may dynamically measure the four axial force components along the axial direction on both sides of the ultrasonic probe 1341, so that the controller 140 may calculate the first axial force based on the four axial force components, and then calculate the second axial force on the ultrasonic probe 1341 along the central axial direction and the normal pressure of the ultrasonic probe 1341 on the skin. It should be noted that the force sensing module 133 may include a plurality of one-dimensional sensors 1331 so as to obtain a plurality of axial force components when the ultrasonic probe 1341 fits the human body. It is understandable that the number of one-dimensional sensors 1331 used in the force sensing module 133 may be set according to actual design requirements, and the present disclosure does not impose any specific limitation on this.

As shown in FIG. 6, the force sensing module 133 includes a six-dimensional sensor 1332, which is used to replace the two one-dimensional sensors 1331. A six-dimensional sensor 1332 may also be used to obtain the axial force components on both sides of the ultrasonic probe 1341 when the ultrasonic probe 1341 fits the human body.

Those having ordinary skill in the art will understand that the system structure shown in the diagrams does not constitute a limitation on the embodiments of the present disclosure, and may include more or fewer components than shown in the Figure, or combine certain components, or arrange the components differently.

The system embodiments described above are merely illustrative, where the units described as separate components may or may not be physically separated, that is, they may be located in one place or distributed across a plurality of network units. Some or all of the modules may be selected according to actual needs to achieve the purpose of the solution in this embodiment.

Those having ordinary skill in the art will understand that the system architecture and application scenarios described in the embodiments of the present disclosure are intended to more clearly illustrate the technical solutions of the embodiments of the present disclosure, and do not constitute a limitation on the technical solutions provided by the embodiments of the present disclosure. Those having ordinary skill in the art will know that with the evolution of the system architecture and the emergence of new application scenarios, the technical solutions provided by the embodiments of the present disclosure are equally applicable to similar technical problems.

Based on the above system architecture and device structure, various embodiments of a method for controlling the puncture operation robot of the present disclosure are proposed below.

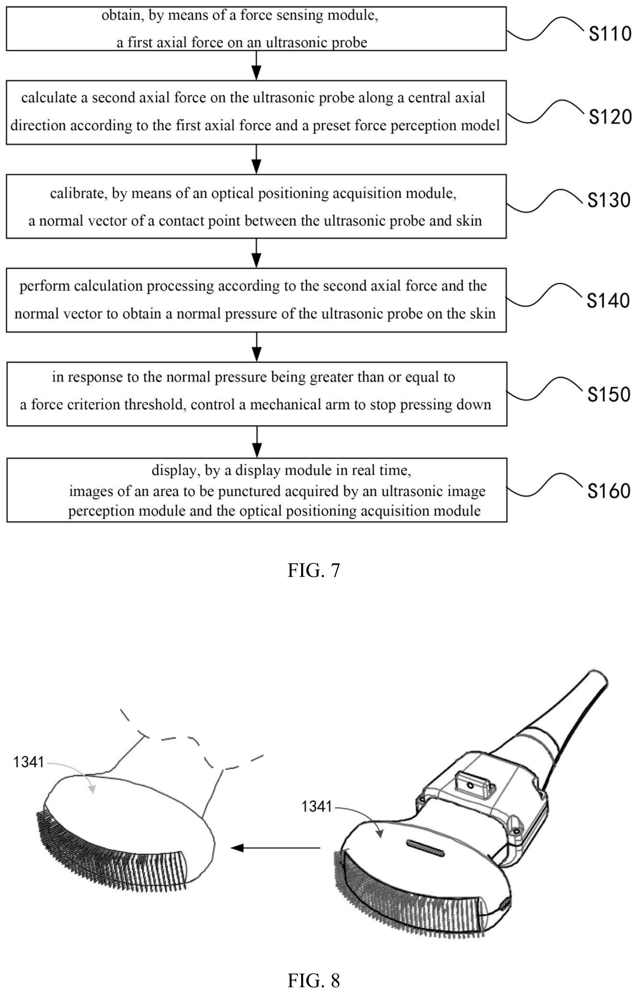

As shown in FIG. 7, a method for controlling the puncture operation robot may be applied to the system architecture of the puncture operation robot shown in FIG. 1. The method for controlling the puncture operation robot may include but is not limited to steps S110 to S160.

In a step of S110: The force sensing module obtains a first axial force on the ultrasonic probe.

In this step, when it is impossible to directly obtain the force on the ultrasonic probe along the central axial direction, the first axial force on the ultrasonic probe along the central axial direction is indirectly obtained through the force sensing module, which is helpful in providing a data basis for the subsequent calculation of the second axial force and normal pressure on the ultrasonic probe along the central axial direction.

In accordance with some embodiments of the present disclosure, the force sensing module includes at least two one-dimensional sensors; the obtaining, by means of the force sensing module, the first axial force on the ultrasonic probe includes: obtaining, by means of the at least two one-dimensional sensors, at least two axial force components; and obtaining, by means of an addition of the at least two axial force components, the first axial force.

In one embodiment, in a case where the force sensing module includes two one-dimensional sensors and when the ultrasonic probe fits the human body, the two one-dimensional sensors may dynamically measure two axial force components $\vec{f}_1$ and $\vec{f}_2$ along the axial direction on both sides of the ultrasonic probe, so that the controller may calculate the first axial force $\vec{F}_1$ based on the two axial force components $\vec{f}_1$ and $\vec{f}_2$. In one embodiment, in a case where the force sensing module includes two one-dimensional sensors, the first axial force $\vec{F}_1$ is:

$$\vec{F}_1 = \vec{f}_1 + \vec{f}_2.$$

In one embodiment, in a case where the force sensing module includes four one-dimensional sensors and when the ultrasonic probe fits the human body, the four one-dimensional sensors may dynamically measure four axial force components $\vec{f}_1$, $\vec{f}_2$, $\vec{f}_3$ and $\vec{f}_4$ along the axial direction on both sides of the ultrasonic probe, so that the controller may calculate the first axial force $\vec{F}_1$ based on the four axial force components. In one embodiment, in a case where the force sensing module includes four one-dimensional sensors, the first axial force $\vec{F}_1$ is:

$$\vec{F}_1 = \vec{f}_1 + \vec{f}_2 + \vec{f}_3 + \vec{f}_4.$$

In one embodiment, in a case where the force sensing module includes a six-dimensional sensor or a plurality of one-dimensional sensors and when the ultrasonic probe fits the human body, the six-dimensional sensor or the plurality of one-dimensional sensors may dynamically measure a plurality of axial force components of the ultrasonic probe along the axial direction. Similarly, the first axial force $\vec{F}_1$ may be obtained by calculation based on the plurality of axial force components. The specific calculation method is the same as the above method and will not be repeated here.

In a step of S120: a second axial force on the ultrasonic probe along a central axial direction is calculated according to the first axial force and a preset force perception model.

In this step, the force on the ultrasonic probe along the central axial direction cannot be directly obtained. Although the first axial force on the ultrasonic probe along the central axial direction is indirectly obtained through the force sensing module, the actual axial force on the ultrasonic probe along the central axial direction deviates from the first axial force due to factors such as the material and size of the probe. Therefore, it is necessary to set a preset force perception model, input the first axial force into the preset force perception model, and output the second axial force. It may be understood that the second axial force, obtained after further optimization of the preset force perception model, is closer to the actual force on the ultrasonic probe along the central axial direction, making it helpful to serve as reliable reference data for subsequent judgment on whether the mechanical arm needs to be stopped, thereby improving the accuracy and safety of a puncture operation.

In accordance with some embodiments of the present disclosure, step S120 includes: setting the preset force perception model to: $\vec{f}_s = k * \vec{F}_1$, where $\vec{F}_1$ is the first axial force, $\vec{f}_s$ is the second axial force, and k is a constant coefficient; and inputting the measured first axial force into the preset force perception model for calculation, and outputting to obtain the second axial force.

Since the ultrasonic probe is made of a uniform material, the stress changes linearly across its cross-section, leading to $\vec{f}_s = k * \vec{F}_1$, where k is the constant coefficient that is related to the probe material and size. In order to simplify the preset force perception model, k may be assigned a value of 1. When k=1, the preset force perception model is: $\vec{f}_s = \vec{F}_1$. It is understandable that the constant coefficient k may be assigned a value according to the actual probe material and size. Therefore, the present disclosure does not impose any specific limitation on the value of the constant coefficient k.

In one embodiment, when the force sensing module includes two one-dimensional sensors, two axial force components $\vec{f}_1$ and $\vec{f}_2$ along the axial direction on both sides of the ultrasonic probe are measured, and the first axial force $\vec{F}_1$ is calculated as: $\vec{F}_1 = \vec{f}_1 + \vec{f}_2$; the second axial force $\vec{f}_s$ at this time is:

$$\vec{f}_s = k * (\vec{f}_1 + \vec{f}_2).$$

In one embodiment, in a case where the force sensing module includes four one-dimensional sensors, four axial force components $\vec{f}_1$, $\vec{f}_2$, $\vec{f}_3$ and $\vec{f}_4$ along the axial direction on both sides of the ultrasonic probe are measured, and the first axial force $\vec{F}_1$ is calculated as: $\vec{F}_1 = \vec{f}_1 + \vec{f}_2 + \vec{f}_3 + \vec{f}_4$; the second axial force $\vec{f}_s$ at this time is:

$$\vec{f}_s = k * (\vec{f}_1 + \vec{f}_2 + \vec{f}_3 + \vec{f}_4).$$

In one embodiment, in a case where the force sensing module includes a six-dimensional sensor or a plurality of one-dimensional sensors, the first axial force $\vec{F}_1$ may be obtained by calculation based on the acquired plurality of axial force components, and then the second axial force may be obtained by calculation based on the preset force perception model. The specific calculation method is the same as the above method and will not be repeated here.

Step S130: a normal vector of a contact point between the ultrasonic probe and skin is calibrated by an optical positioning acquisition module.

In this step, the distal end of the ultrasonic probe is an arc curved surface. When the ultrasonic probe fits the human body, the arc curved surface of the ultrasonic probe will be subjected to pressure in all directions. The normal vector $\vec{n}$ of the contact point between the ultrasonic probe and the skin is calibrated to obtain a first angle $\theta$ between the second axial force $\vec{f}_s$ and the normal vector n, which facilitates the subsequent calculation of the positive pressure (i.e., normal pressure) when the ultrasonic probe fits the human skin, and is beneficial to improving the accuracy of the puncture operation.

In one embodiment, step S130 is further described with reference to FIG. 8. In one embodiment, in order to determine the normal vector $\vec{n}$ of the contact point between the ultrasonic probe and the human skin, the arc curved surface at the distal end of the ultrasonic probe is divided in a form of grids. The intersection points of the grids are discrete points. The smaller the grid, the denser the discrete points. Based on this grid division method, the arc curved surface at the distal end of the ultrasonic probe is discretized into a plurality of dense discrete points. The spatial coordinates of each point at the distal end may be obtained through the optical positioning acquisition module, so that the normal vector $\vec{n}$ of each discrete point may be calibrated.

In a step of S140: a normal pressure of the ultrasonic probe on the skin is calculated using the second axial force and the normal vector.

In this step, a module value $|\vec{f}_n|$ of the normal pressure of the ultrasonic probe on the skin is obtained according to the second axial force $\vec{f}_s$ obtained by calculation and the normal vector $n$ obtained by calibration of the optical positioning acquisition module. The normal pressure reflects the degree of fit between the ultrasonic probe and the skin.

In a step of S150: in response to the normal pressure being greater than or equal to a force criterion threshold, the mechanical arm is controlled to stop pressing down.

In this step, the normal pressure $|\vec{f}_n|$ is compared with the force criterion threshold F. In response to the normal pressure $|\vec{f}_n|$ being greater than or equal to the force criterion threshold F, the mechanical arm is controlled to stop pressing down, and thus the probe stops pressing down, indicating that the normal pressure is appropriate at this time. Simultaneously, the mechanical arm is controlled to stop pressing down to remind the doctor that the normal pressure is appropriate and the next manual operation can be performed. This effectively reduces the probability of two situations: the normal pressure of fitting is too small, causing blurred ultrasonic images; and the pressure is too large, causing severe compression and deformation of the patient's organs, thereby improving the operation efficiency, operation accuracy and safety of the semi-automatic puncture operation.

According to some embodiments of the present disclosure, the control method further includes: when the normal pressure is smaller than the force criterion threshold, controlling the mechanical arm to keep pressing down, where the probe keeps pressing down. This realizes partial automated control, which is beneficial to improving the operation efficiency of the semi-automatic puncture operation.

It is understandable that the force criterion threshold F may be set to 255.

In one embodiment, the force criterion threshold F may be tested through multiple experiments. Under the premise of ensuring the clarity of ultrasonic images and the comfort of the human body, experimental tests determine the force criterion threshold F and determine the mechanical arm operation judgment criteria based on force perception: $|\vec{f}_n| \geq F$, then the probe is controlled to stop pressing down; otherwise, $|\vec{f}_n| < F$, the probe is controlled to keep pressing down.

In a step of S160: images of an area to be punctured that are acquired by the ultrasonic image perception module and the optical positioning acquisition module is displayed by means of the display module in real time.

In this step, after the images of the area to be punctured are acquired by the ultrasonic image perception module and the optical positioning acquisition module, the images of the area to be punctured are displayed in real time on the display module, which is convenient for the doctor to complete a puncture action by adjusting and operating the puncture mechanism based on the displayed images, thereby improving the operation accuracy and safety of the puncture operation.

In one embodiment, the images of the area to be punctured includes a lesion image and a position reference image.

In accordance with some embodiments of the present disclosure, step S160 includes: acquiring an ultrasonic image of the skin through the ultrasonic image perception module; performing image processing on the ultrasonic image to obtain the lesion image; obtaining a needle thrusting path of a puncture distal end of the puncture mechanism through the optical positioning acquisition module; and displaying, by means of the display module in real time, the lesion image and the position reference image, where the position reference image is used for displaying a positional relationship between the needle thrusting path and a lesion on the skin.

In the embodiments of the present disclosure, by adopting the method for controlling the puncture operation robot including above steps S110 to S160, during a semi-automatic puncture operation, the puncture operation robot first obtains, by means of the force sensing module, the first axial force on the ultrasonic probe; next, the second axial force on the ultrasonic probe along the central axial direction is calculated according to the first axial force and the preset force perception model; then, the normal vector of the contact point between the ultrasonic probe and the skin is calibrated by means of the optical positioning acquisition module; next, calculation processing is performed according to the second axial force and the normal vector to obtain the normal pressure of the ultrasonic probe on the skin; finally, in response to the normal pressure being greater than or equal to the force criterion threshold, the mechanical arm is controlled to stop pressing down, thereby prompting a doctor that the normal pressure generated by fitting of the ultrasonic probe and the patient's skin is appropriate, which effectively reduces the probability of two situations: the normal pressure of fitting is too small, causing blurred ultrasonic images; and the pressure is too large, causing severe compression and deformation of the patient's organs. Simultaneously, the images of the area to be punctured that are acquired by the ultrasonic image perception module and the optical positioning acquisition module are displayed by means of the display module in real time, so that the doctor can complete a puncture action by adjusting and operating the puncture mechanism based on the displayed images, thus achieving the semi-automatic puncture operation with enhanced efficiency, accuracy, and safety. That is to say, the embodiments of the present disclosure may provide doctors with reliable operation reference information by utilizing the puncture operation robot, thereby improving the operation efficiency, operation accuracy and safety when the semi-automatic puncture operation is performed by using the puncture operation robot.

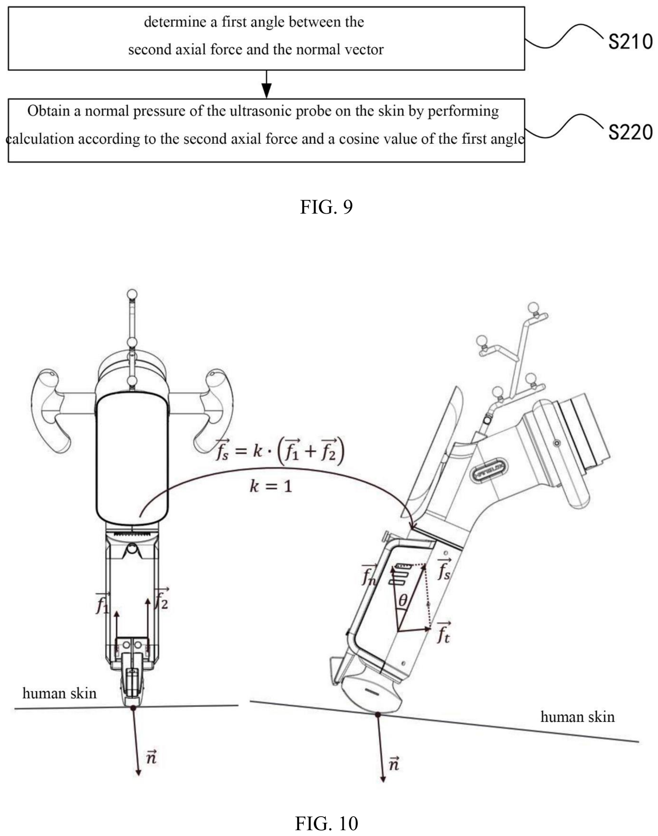

Referring to FIG. 9, in accordance with some embodiments of the present disclosure, step S140 may include but is not limited to steps S210 to S220.

In a step of S210: a first angle between the second axial force and the normal vector is determined; and

In a step of S220: a normal pressure of the ultrasonic probe on the skin is derived from the second axial force and a cosine value of the first angle.

The normal pressure is calculated through steps S210 to S220, providing a control basis for automatically controlling the operation of the mechanical arm.

In one embodiment, after determining the first angle $\theta$ between the second axial force and the normal vector, the module value $|\vec{f}_s|$ of the second axial force is calculated, and the normal pressure value is obtained by multiplying the module value $|\vec{f}_s|$ of the second axial force by the cosine value of the first angle $\theta$. In other words, the calculation formula for the normal pressure of the ultrasonic probe on the skin is: $|\vec{f}_n| = |\vec{f}_s|\, cos\, \theta$.

In one embodiment, as shown in FIG. 10, in a case where the force sensing module includes two one-dimensional sensors, it is assumed that at time t, the ultrasonic probe 1341 initially contacts the human skin, and the normal vector $\vec{n}$ is calibrated at the contact point. Since the ultrasonic probe itself has a certain weight, the sensor will be subjected to a certain pulling force $\vec{f}_t$, thus, $\vec{f}_1, \vec{f}_2$ and $\vec{f}_s$ are directional here. The force analysis of the ultrasonic probe and the two sensors is shown in FIG. 10. Therefore, according to Newton's law, the normal pressure at the contact point may be obtained as $|\vec{f}_n| = |\vec{f}_s|\, cos\, \theta$, where the first angle $\theta$ is the angle between $\vec{f}_s$ and $\vec{n}$. Since $|\vec{f}_n|$ and $\vec{n}$ are parallel, the first angle $\theta$ is also the angle between $\vec{f}_s$ and $|\vec{f}_n|$.

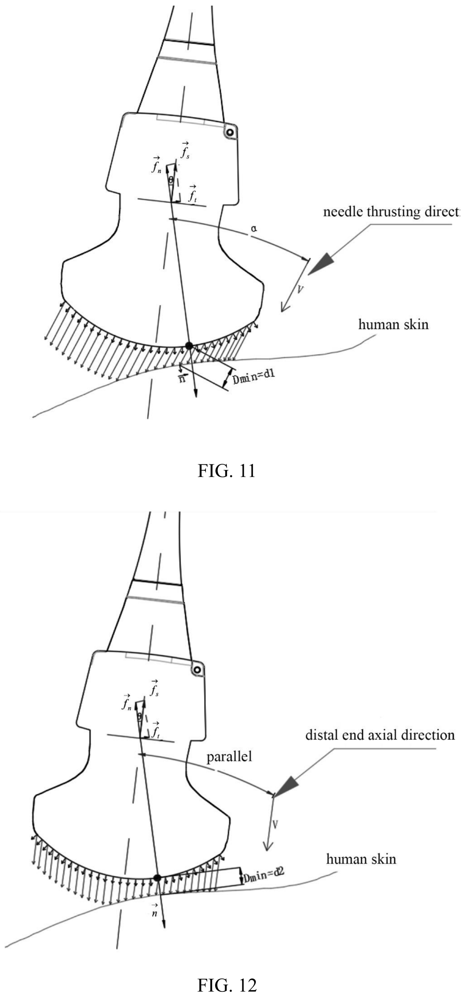

As shown in FIG. 11 and FIG. 12, at time $t - \delta t$, the press-down speed $\vec{v}$ of the ultrasonic probe may be provided by a puncture operation robot system (it may be selected whether the puncture distal end moves along the needle thrusting direction or the distal end axis when approaching the human body). A human skin surface model may be provided by a digital human body established before the operation through methods such as Maker small ball optical positioning. Therefore, the point closet to the human skin for each discrete point to reach along the direction of the press-down speed $\vec{v}$ of the probe may be calculated. Assuming that the discrete points are dense enough, the point closest to the skin is the first discrete point to contact the skin during the actual down pressure process.

If the mechanical distal end moves along the needle thrusting direction when approaching the human body, then there is a second angle $\alpha$ between the motion direction and the distal end axis, and the shortest distance between the ultrasonic probe and the skin may be calculated as $D_{min}=d_1$ (as shown in FIG. 11). If the mechanical distal end moves along the distal end axis when approaching the human body, then the motion direction is parallel to the distal end axis, and the shortest distance between the ultrasonic probe and the skin is $D_{min}=d_2$ (as shown in FIG. 12). The corresponding discrete point of the probe is the contact point at time t, and thus the normal of the discrete point of the probe is the normal $\vec{n}$ of the contact point. Finally, the first angle $\theta$ is obtained by calculating the angle between the axial direction ($\vec{f}_s$ direction) of the ultrasonic probe and the normal direction (n direction) of the contact point.

In summary, the normal pressure $\vec{f}_n$ at the contact point may be obtained as:

$$\left|\vec{f_n}\right| = \left|\vec{f_1} + \vec{f_2}\right| cos\, \theta.$$

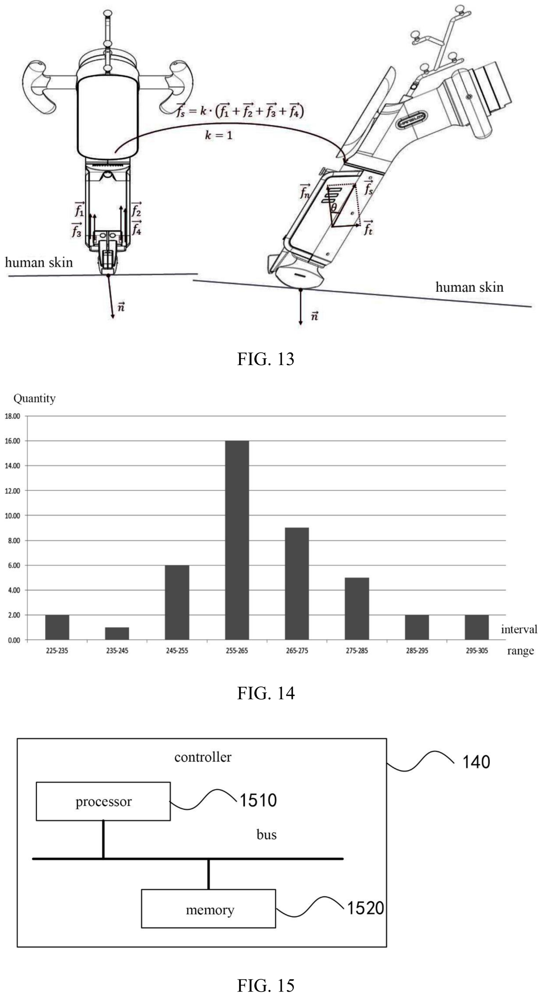

As shown in FIG. 13, in a case where the force sensing module includes four one-dimensional sensors, the force analysis of the ultrasonic probe and the four sensors is shown in FIG. 13. Therefore, according to Newton's theorem, the normal pressure at the contact point may be obtained:

$$\left|\vec{f_n}\right| = \left|\vec{f_s}\right| cos\, \theta = \left|k(\vec{f_1} + \vec{f_2} + \vec{f_3} + \vec{f_4})\right| cos\, \theta\,, k = 1.$$

Where: the first angle $\theta$ is the angle between $\vec{f}_s$ and $\vec{n}$, and is also the angle between $\vec{f}_s$ and $\vec{f}_n$. When there are a plurality of one-dimensional sensors, the method for calculating the normal pressure is as shown above and will not be repeated here.

An example is given to illustrate the specific implementation method for the experimental test of the force criterion threshold F.

A body membrane is used to simulate the human body. Six lesion points are set up at different locations within the body membrane. Three different needle thrusting paths are planned for each lesion point. The puncture operation robot is controlled to move closer to the body membrane in the direction of needle thrusting or the distal end axis. Finally, the puncture operation robot is fine-tuned to ensure that the distal-end ultrasonic probe fits the human skin. It is observed with the naked eyes: if the ultrasound is clearly visible, and the lesion inside the body membrane and the ultrasonic probe do not squeeze or deform the body membrane, the fitting pressure is appropriate. In the case where the force sensing module includes two one-dimensional sensors, values $\vec{f}_1$ and $\vec{f}_2$ of the two one-dimensional sensors and the first angle $\theta$ between the second axial force $\vec{f}_s$ and the normal vector n are recorded, where, $\vec{f}_1$ and $\vec{f}_2$ represent the differences in the readings from the two respective sensors between the mechanical arm's hovering state and its state after pressing down against the body membrane. The hovering state of the mechanical arm refers to a state where the mechanical arm comes to a stop above the body membrane after moving according to the planned path, and is poised to further move in the direction of needing thrusting or the distal end axial direction to fit the body membrane. The process of solving the value of the first angle $\theta$ has been clearly described above and may be directly read out through software calculation. The values $\vec{f}_1$ and $\vec{f}_2$, and the first angle $\theta$ are substituted into the formula: $|\vec{f}_n| = |\vec{f}_1 + \vec{f}_2|\cos\theta$, thus obtaining $|\vec{f}_n|$. After multiple measurements, a plurality of data $|\vec{f}_n|$ may be obtained. The plurality of data $|\vec{f}_n|$ are organized into a quantity-interval value statistical distribution diagram as shown in FIG. 14. As shown FIG. 14, the value of $|\vec{f}_n|$ occupies the largest proportion in the range of 255-265, so the force criterion threshold is 255. Then, the conditional statement "if $|\vec{f}_n| = |\vec{f}_1 + \vec{f}_2|\cos\theta \geq 255$, then the probe stops pressing down" is incorporated as a decision criterion into the controller's program for controlling the mechanical arm, and the above experiment is repeated. But this time, the force feedback signal is detected by software to control the mechanical arm to stop automatically, without manual fine-tuning any longer. After stopping, the clarity of the ultrasonic probe and the extent of skin compression are observed to further verify the correctness of the force criterion threshold. The force criterion threshold is finally determined to be 255. It is understandable that in a case where the force sensing module includes four or a plurality of one-dimensional sensors or one six-dimensional sensor, the plurality of $|\vec{f}_n|$ may be calculated in the same manner as above, thereby determining the force criterion threshold, which is not repeated here.

Referring to FIG. 15, the controller 140 includes: a memory 1520, a processor 1510 and a computer program stored in the memory 1520 and executable by the processor, wherein the computer program, when executed by the processor 1510, causes the processor 1510 to perform the above-mentioned method for controlling the puncture operation robot.

The processor 1510 and the memory 1520 may be connected via a bus or other means.

The processor 1510 may be implemented as a general-purpose central processing unit, a microprocessor, an application-specific integrated circuit, or one or more integrated circuits, and is used to execute relevant programs to implement the technical solutions provided by the embodiments of the present disclosure.

The memory 1520, as a non-transitory computer-readable storage medium, may be used to store non-transitory software programs and non-transitory computer-executable programs. In addition, the memory may include high-speed random access memory and may also include non-volatile memory, such as at least one disk storage device, flash memory device, or other non-volatile solid-state storage device. In some implementations, the memory 1520 may include a memory located remotely from the processor, and these remote memories may be connected to the processor via a network. Examples of the aforementioned network include, but are not limited to, the Internet, an intranet, a local area network, a mobile communication network, and combinations thereof.

The non-transient software program and instructions required to implement the method for controlling the puncture operation robot in the above embodiment are stored in the memory. When the program and instructions are executed by the processor, the method for controlling the puncture operation robot in the above embodiment is executed, for example, the method steps shown in FIG. 7 and FIG. 9 described above are executed.

An embodiment of the present disclosure further provides a computer-readable storage medium, which stores computer-executable instructions. The computer-executable instructions are executed by a processor or controller, for example, by a processor in the above-mentioned device embodiment, so that the above-mentioned processor may execute the method for controlling the puncture operation robot in the above-mentioned embodiment, for example, execute the method steps shown in FIG. 7 and FIG. 9 described above.

Those of ordinary skill in the art will understand that all or some of the steps and systems in the methods disclosed above may be implemented as software, firmware, hardware, or a suitable combination thereof. Some or all of the physical components may be implemented as software executed by a processor, such as a central processing unit, a digital signal processor or a microprocessor, or as hardware, or as an integrated circuit, such as an application specific integrated circuit. Such software may be distributed on computer-readable media, which may include computer storage media (or non-transitory media) and communication media (or transitory media). As known to those of ordinary skill in the art, the term computer storage media includes volatile and nonvolatile, removable and non-removable media implemented in any method or technology for storage of information such as computer-readable instructions, data structures, program modules or other data. The computer storage medium includes, but is not limited to, RAM, ROM, EEPROM, flash memory or other memory technology, CD-ROM, digital versatile disks (DVD) or other optical disk storage, magnetic cassettes, magnetic tape, magnetic disk storage or other magnetic storage devices, or any other medium that is capable of storing desired information and accessible by a computer. Further, as is well known to those of ordinary skill in the art, a communication medium typically includes computer-readable instructions, data structures, program modules, or other data in a modulated data signal such as a carrier wave or other transport mechanism, and may include any information delivery media.

The above is a specific description of the preferred implementation of the present disclosure, but the present disclosure is not limited to the above implementation. Those of ordinary skill in the art can make various equivalent modifications or substitutions without violating the scope of the present disclosure. These equivalent modifications or substitutions are all included in the scope defined by the present disclosure.

**Claims**

1. A puncture operation robot, comprising:

   a mechanical arm;
   a puncture mechanism arranged at a distal end of the mechanical arm;
   a puncture perception device arranged on the puncture mechanism, the puncture perception device comprises a display module, an optical positioning acquisition module, a force sensing module, and an ultrasonic image perception module comprising an ultrasonic probe; and
   a controller, the controller is electrically connected to the mechanical arm, the display module, the optical positioning acquisition module, the ultrasonic image perception module and the force sensing module, respectively.

2. The puncture operation robot according to claim 1, wherein the force sensing module comprises at least two one-dimensional sensors.

3. A method for controlling a puncture operation robot, applied to the puncture operation robot according to any one of claims 1 to 2, wherein the puncture operation robot comprises: a mechanical arm; a puncture mechanism arranged at a distal end of the mechanical arm; a puncture perception device arranged on the puncture mechanism, the puncture perception device comprises a display module, an optical positioning acquisition module, a force sensing module, and an ultrasonic image perception module comprising an ultrasonic probe; and a controller, the controller is electrically connected to the mechanical arm, the display module, the optical positioning acquisition module, the ultrasonic image perception module and the force sensing module, respectively; and
   wherein the control method comprises:

   obtaining, by the force sensing module, a first axial force on the ultrasonic probe;
   calculating a second axial force on the ultrasonic probe along a central axial direction based on the first axial force and a preset force perception model;
   calibrating, by the optical positioning acquisition module, a normal vector of a contact point between the ultrasonic probe and skin;
   performing calculation processing based on the second axial force and the normal vector to obtain a normal pressure of the ultrasonic probe on the skin;
   in response to the normal pressure being greater than or equal to a force criterion threshold, controlling the mechanical arm to stop pressing down; and
   displaying, by the display module in real time, images of an area to be punctured acquired by the ultrasonic image perception module and the optical positioning acquisition module.

4. The control method according to claim 3, wherein the force sensing module comprises at least two one-dimensional sensors; the obtaining, by the force sensing module, a first axial force on the ultrasonic probe comprises:

   obtaining, by the at least two one-dimensional sensors, at least two axial force components; and
   obtaining, by summing the at least two axial force components, the first axial force.

5. The control method according to claim 3, wherein the calculating a second axial force on the ultrasonic probe along a central axial direction based on the first axial force and a preset force perception model comprises:

   setting the preset force perception model to be $\vec{f}_s = k * \vec{F}_1$, wherein $\vec{F}_1$ is the first axial force, $\vec{f}_s$ is the second axial force, and k is a constant coefficient; and
   inputting the measured first axial force into the preset force perception model for calculation to obtain the second axial force.

6. The control method according to claim 3, wherein the performing calculation processing based on the second axial force and the normal vector to obtain a normal pressure of the ultrasonic probe on the skin comprises:

   determining a first angle between the second axial force and the normal vector; and

obtaining the normal pressure of the ultrasonic probe on the skin by performing calculation based on the second axial force and a cosine value of the first angle.

7. The control method according to claim 3, wherein the images of the area to be punctured comprise a lesion image and a position reference image; and the displaying, by the display module in real time, images of an area to be punctured acquired by the ultrasonic image perception module and the optical positioning acquisition module comprises:

   acquiring an ultrasonic image of the skin through the ultrasonic image perception module;
   performing image processing on the ultrasonic image to obtain the lesion image;
   obtaining a needle thrusting path of a puncture distal end of the puncture mechanism through the optical positioning acquisition module; and
   displaying, by the display module in real time, the lesion image and the position reference image, the position reference image is used for displaying a positional relationship between the needle thrusting path and a lesion on the skin.

8. The control method according to claim 3, further comprising: in response to the normal pressure being smaller than the force criterion threshold, controlling the mechanical arm to keep pressing down.

9. A controller, comprising: a memory, a processor, and a computer program stored in the memory and executable by the processor, wherein the computer program, when executed by the processor, causes the processor to perform the method for controlling the puncture operation robot according to any one of claims 3 to 7.

10. A computer-readable storage medium storing computer-executable instructions, wherein the computer-executable instructions are configured to enable a computer to perform the method for controlling the puncture operation robot according to any one of claims 3 to 7.

100

puncture operation robot

110
mechanical arm

140
controller

120
puncture mechanism

130
puncture perception device

Electric connection

131
display module

132
optical positioning acquisition module

133
force sensing module

134
ultrasonic image perception module

1341
ultrasonic probe

FIG. 1

130

1311

1331     1331

FIG. 2

FIG. 3

FIG. 4

1331

FIG. 5

1332

FIG. 6

obtain, by means of a force sensing module, a first axial force on an ultrasonic probe — S110

calculate a second axial force on the ultrasonic probe along a central axial direction according to the first axial force and a preset force perception model — S120

calibrate, by means of an optical positioning acquisition module, a normal vector of a contact point between the ultrasonic probe and skin — S130

perform calculation processing according to the second axial force and the normal vector to obtain a normal pressure of the ultrasonic probe on the skin — S140

in response to the normal pressure being greater than or equal to a force criterion threshold, control a mechanical arm to stop pressing down — S150

display, by a display module in real time, images of an area to be punctured acquired by an ultrasonic image perception module and the optical positioning acquisition module — S160

FIG. 7

FIG. 8

| | |
|---|---|
| determine a first angle between the second axial force and the normal vector | S210 |
| Obtain a normal pressure of the ultrasonic probe on the skin by performing calculation according to the second axial force and a cosine value of the first angle | S220 |

FIG. 9

$$\vec{f_s} = k \cdot \left(\vec{f_1} + \vec{f_2}\right)$$
$$k = 1$$

FIG. 10

FIG. 11

FIG. 12

$$\vec{f_s} = k \cdot (\vec{f_1} + \vec{f_2} + \vec{f_3} + \vec{f_4})$$

$$k = 1$$

FIG. 13

FIG. 14

FIG. 15

## INTERNATIONAL SEARCH REPORT

| International application No. |
|---|
| **PCT/CN2023/114248** |

### A. CLASSIFICATION OF SUBJECT MATTER

A61B34/30(2016.01)i

According to International Patent Classification (IPC) or to both national classification and IPC

### B. FIELDS SEARCHED

Minimum documentation searched (classification system followed by classification symbols)

IPC8: A61B34, A61B17, A61B8

Documentation searched other than minimum documentation to the extent that such documents are included in the fields searched

Electronic data base consulted during the international search (name of data base and, where practicable, search terms used)

CNTXT, ENTXTC, VEN, ENTXT, CNKI: 超声, 探头, 扇, 轴, 轴向, 压力, 法向, 垂直, 垂向, 法线, 皮肤, 表皮, 力, 传感, 感测, 探测, 测量, 监测, 检测 Ultrasound, Probe, Sector, Axis, Axial, Pressure, Normal, Vertical, Skin, Force, Sensing, Detecting, Measuring, Monitoring

### C. DOCUMENTS CONSIDERED TO BE RELEVANT

| Category* | Citation of document, with indication, where appropriate, of the relevant passages | Relevant to claim No. |
|---|---|---|
| PX | CN 116725686 A (ZHUHAI HANGLOK MEDICAL TECHNOLOGY CO., LTD.) 12 September 2023 (2023-09-12) <br> description, paragraphs [0001]-[0132], claims 1-10, and figures 1-15 | 1-10 |
| PX | CN 117204953 A (ZHUHAI HANGLOK MEDICAL TECHNOLOGY CO., LTD.) 12 December 2023 (2023-12-12) <br> description, paragraphs [0053]-[0067] and [0081]-[0085], claims 1 and 6, and figures 1-3 | 1-2 |
| X | CN 114767265 A (ZHUHAI HANGLOK MEDICAL TECHNOLOGY CO., LTD.) 22 July 2022 (2022-07-22) <br> description, paragraphs [0004] and [0022]-[0075] | 1-2 |
| A | CN 111973225 A (KONINKLIJKE PHILIPS N.V.) 24 November 2020 (2020-11-24) <br> entire document | 1-10 |
| A | CN 112472133 A (SHENZHEN DELICA MEDICAL EQUIPMENT CO., LTD.) 12 March 2021 (2021-03-12) <br> entire document | 1-10 |

☑ Further documents are listed in the continuation of Box C.　　☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **09 January 2024** | **10 January 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/114248** |

**C.** **DOCUMENTS CONSIDERED TO BE RELEVANT**

| Category* | Citation of document, with indication, where appropriate, of the relevant passages | Relevant to claim No. |
| --- | --- | --- |
| A | CN 112656442 A (SHENZHEN DELICA MEDICAL EQUIPMENT CO., LTD.) 16 April 2021 (2021-04-16)<br>entire document | 1-10 |
| A | CN 115670675 A (WUHAN UNIVERSITY) 03 February 2023 (2023-02-03)<br>entire document | 1-10 |
| A | CN 217723536 U (MUDANJIANG MEDICAL UNIVERSITY) 04 November 2022 (2022-11-04)<br>entire document | 1-10 |
| A | US 2020015780 A1 (KONINKLIJKE PHILIPS N.V.) 16 January 2020 (2020-01-16)<br>entire document | 1-10 |
| A | US 2021077069 A1 (GE PRECISION HEALTHCARE LLC.) 18 March 2021 (2021-03-18)<br>entire document | 1-10 |
| A | US 2021378644 A1 (JOHNS HOPKINS UNIVERSITY) 09 December 2021 (2021-12-09)<br>entire document | 1-10 |
| A | US 2022054201 A1 (VITESTRO HOLDING B.V.) 24 February 2022 (2022-02-24)<br>entire document | 1-10 |

Form PCT/ISA/210 (second sheet) (July 2022)

## INTERNATIONAL SEARCH REPORT
### Information on patent family members

| International application No. |
| --- |
| **PCT/CN2023/114248** |

| Patent document cited in search report | | | Publication date (day/month/year) | Patent family member(s) | | | Publication date (day/month/year) |
| --- | --- | --- | --- | --- | --- | --- | --- |
| CN | 116725686 | A | 12 September 2023 | None | | | |
| CN | 117204953 | A | 12 December 2023 | None | | | |
| CN | 114767265 | A | 22 July 2022 | None | | | |
| CN | 111973225 | A | 24 November 2020 | WO | 2015101949 | A1 | 09 July 2015 |
| | | | | US | 2016324501 | A1 | 10 November 2016 |
| | | | | US | 11096656 | B2 | 24 August 2021 |
| | | | | JP | 2019134958 | A | 15 August 2019 |
| | | | | JP | 7098565 | B2 | 11 July 2022 |
| | | | | EP | 3508134 | A1 | 10 July 2019 |
| | | | | EP | 3508134 | B1 | 04 November 2020 |
| | | | | RU | 2016131480 | A | 07 February 2018 |
| | | | | RU | 2016131480 | A3 | 03 August 2018 |
| | | | | RU | 2689176 | C2 | 24 May 2019 |
| | | | | EP | 3089671 | A1 | 09 November 2016 |
| | | | | EP | 3089671 | B1 | 22 May 2019 |
| | | | | JP | 2021062222 | A | 22 April 2021 |
| | | | | JP | 7165181 | B2 | 02 November 2022 |
| | | | | US | 2021353250 | A1 | 18 November 2021 |
| | | | | EP | 3760129 | A1 | 06 January 2021 |
| | | | | JP | 2017500955 | A | 12 January 2017 |
| | | | | JP | 6517817 | B2 | 22 May 2019 |
| CN | 112472133 | A | 12 March 2021 | None | | | |
| CN | 112656442 | A | 16 April 2021 | None | | | |
| CN | 115670675 | A | 03 February 2023 | None | | | |
| CN | 217723536 | U | 04 November 2022 | None | | | |
| US | 2020015780 | A1 | 16 January 2020 | EP | 3369381 | A1 | 05 September 2018 |
| | | | | US | 11272902 | B2 | 15 March 2022 |
| | | | | WO | 2018158369 | A1 | 07 September 2018 |
| | | | | EP | 3589210 | A1 | 08 January 2020 |
| | | | | EP | 3589210 | B1 | 05 August 2020 |
| US | 2021077069 | A1 | 18 March 2021 | US | 11446002 | B2 | 20 September 2022 |
| US | 2021378644 | A1 | 09 December 2021 | WO | 2020117783 | A1 | 11 June 2020 |
| US | 2022054201 | A1 | 24 February 2022 | WO | 2020145822 | A1 | 16 July 2020 |
| | | | | EP | 3908192 | A1 | 17 November 2021 |
| | | | | NL | 2022351 | B1 | 13 August 2020 |

Form PCT/ISA/210 (patent family annex) (July 2022)